Europäisches Patentamt

European Patent Office

Office européen des brevets

(11) Publication number: **0 375 063 B1**

# EUROPEAN PATENT SPECIFICATION

(45) Date of publication of patent specification: **08.09.93**  (51) Int. Cl.⁵: **A61K 9/18, A61K 9/22**

(21) Application number: **89203243.4**

(22) Date of filing: **18.12.89**

(54) **Granulates for multiparticulate controlled-release oral compositions.**

(30) Priority: **20.12.88 EP 88202983**

(43) Date of publication of application:
**27.06.90 Bulletin 90/26**

(45) Publication of the grant of the patent:
**08.09.93 Bulletin 93/36**

(84) Designated Contracting States:
**AT BE CH DE ES FR GB GR IT LI LU NL SE**

(56) References cited:

CHEMICAL ABSTRACTS, vol. 103, no. 1, 8 July 1985 Columbus, Ohio, USA HASEGAWA A. et al: "Application of solid dispersions of nifedipine with enteric coating agent to prepare a sustained-release dosage form"

CHEMICAL ABSTRACTS, vol. 106, no. 14, 6 April 1987 Columbus, Ohio, USA OHNISHI N. et al: "Preparation of sustained release granules of nifedipine using a solid dispersion system and pharmacokinetic study after oral administration to rab"bits."

CHEMICAL ABSTRACTS, vol. 105, no. 13, 29 September 1986 Columbus, Ohio, USA OHNISHI N.et al: "Preparation of sustained release suppositories of indomethacin using a solid dispersion system and evaluation of bioavailability in rabbits."

(73) Proprietor: **BROCADES PHARMA B.V.**
**Elisabethhof 19 P.O. Box 108**
**NL-2350 AC Leiderdorp(NL)**

(72) Inventor: **Groenendaal, Jan Willem**
**Koornmarkt 4**
**NL-2611 EE Delft(NL)**
Inventor: **Vork, Edoaldus**
**Populierstraat 27**
**NL-2023 SN Haarlem(NL)**
Inventor: **De Ronde, Hendrikus Adrianus**
**Gerardus**
**Adrianalaan 301**
**NL-3053 JA Rotterdam(NL)**

(74) Representative: **Lavy, Uriel et al**
**Gist-Brocades NV Patents and Trademarks**
**Dept. Wateringseweg 1 P.O. Box 1**
**NL-2600 MA Delft (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to granulates for multi-particulate controlled-release oral compositions comprising biologically active substances, targeted to predetermined parts of the intestine and especially to the lower part thereof, and to oral compositions, containing such granulates.

When the active principle of an oral composition (in a single-unit or multiparticulate product) is to be targeted to the intestine, it is conventionally coated by a single or multiple layer of an acid-resistant and/or slowly-releasing compound. Currently a considerable number of substances and mixtures are known for use in such coatings. European Patent Application 0040590 describes the coating of a core containing a therapeutically active substance by a single layer comprising an acid-resistant polymer and a water-insoluble polymer. A multiple-layered coating is described in U.S. Patent 3,431,338, where a drug-containing nucleus is coated successively with an acid soluble, alkali-resistant material, a water-soluble intermediate layer and an outer alkali-soluble, acid-resistant layer. In practice, the coated preparations are often unreliable and besides their production is cumbersome. Therefore, a need exists for improvement of intestinal drug targeting.

A further development is to use the concept of solid dispersion of the active principle in the release-modifying substance as an alternative to coating the active principle with the release-modifying substance. A solid dispersion, which is to be clearly distinguished from simple mechanical mixes, has been defined as a dispersion of one or more active ingredients in an inert excipient at solid state prepared by the melting (fusion), solvent, or melting-solvent method (J.L. Ford, Pharm.Acta Helv. 67,(1986),69). The concept of solid dispersion was introduced by K. Sekiguchi and N. Obi (Chem. Pharm. Bull. 9, (1961) 866), to improve the bio-availability of a poorly water-soluble drug by dispersing it in a water-soluble excipient. The bulk of subsequent publications to date still concerns such enhanced-release products. But it has also been recognized that a similar concept can be applied to sustained-release products. One example is Japanese Patent Application (Kokai) 61,078,733, which discloses an amorphous solid preparation intended to dissolve ir. the intestine, comprising (a) a non-steroid type anti-inflammatory agent such as indomethacin and (b) one or more pH-dependent high molecular compounds, such as a copolymer of methacrylic acid and methyl methacrylate. It is manufactured by dissolving (a) and (b) in a common solvent, removing the solvent and pulverising the solid formed. Only grains falling within the required size are used, which of course involves loss of substance. Besides, when these grains are to be further processed into tablets, they have to be granulated first, a process in the course of which there exists a danger of adversely influencing the glass-like consistency of the solid dispersion, thus defeating its purpose of acid resistance.

The deposition of solid dispersions of the enhanced-release type on water-soluble carrier particles such as lactose, or on particles of calcium hydrogen phosphate which is soluble in gastric fluid has been proposed by K.H. Kim and C.J. Jarowski (J. Pharm. Sci. 66, (1977) 1536) and by J.L. Ford and M.H. Rubinstein (Pharm. Acta Helv. 55(1980)1), respectively.

The deposition of solid dispersions of the acid-resistant type on a water soluble inert core material (sucrose) has been described by A. Hasegawa et al. (Chem. Pharm. Bull. 33 (1985)1615). This method involves a relatively cumbersome procedure of spray-coating the solid dispersion onto the water-soluble sucrose cores, for which a special apparatus is required. The thus coated sucrose particles are spherical and smooth, and they cannot be compressed into tablets without an extra step of granulation. Besides, this method is not suitable for solid dispersions of the release-limiting (pH-independent) type since these solid dispersions will allow already in the acid environment of the stomach the occurrence of aqueous diffusion, which will dissolve the cores, changing the form of the particles and making the release characteristics unpredictable and uncontrollable.

It has now been found that water-insoluble carrier particles do not have the above disadvantages, and that granulates for multiparticulate oral compositions wherein a biologically active compound is incorporated in solid dispersion with an acid-resistant or release-limiting compound can be prepared more efficiently and effectively by mixing this solid dispersion with water-insoluble carrier particles.

When water-insoluble carrier particles are used, they can simply be mixed with the dispersion before it is solidified, without any need to actively deposit the solid on the carrier cores. After the complete mixture has solidified, it is further processed to a granulate according to granulation methods known in the art, such as oscillating-sieving or extrusion.

The process according to the invention is very versatile since it is applicable to both acid-resistant and release-limiting preparations. The process is also very efficient since no special apparatus is required for the simple step of mixing the water-insoluble carrier particles with the dispersion, and since due to the granules being irregular in shape and porous they can be immediately compressed into tablets.

Thus, the present invention provides a method for preparing a granulate for a multiparticulate oral composition based on the concept of solid dispersion, whereby a biologically active substance is dispersed in an acid-resistant or release-limiting substance using the melting, the solvent or the melting-solvent method, characterized in that before the dispersion is solidified it is mixed with water-insoluble carrier particles whereafter the complete mixture is further processed according to granulation methods known in the art.

The invention also provides granulates for multi-particulate oral compositions comprising a biologically active substance in solid dispersion with an acid-resistant and/or a release-limiting compound, characterized in that the solid dispersion is mixed with water-insoluble carrier particles.

It will be appreciated that in principle any biologically active compound can be incorporated in the granulates for multiparticulate oral compositions of this invention, and in particular those compounds, e.g. the therapeutic (poly) peptides, which are sensitive to acid or to digestive enzymes and those which are disagreeable to the stomach, but that the main application of this invention lies with compounds which are meant to act locally in the intestine. Examples of the latter are corticosteroids and non-steroidal anti-inflammatory compounds, especially beclomethasone 17,21-dipropionate and 5- or 4-amino-salicylic acid or their derivatives. Further examples are bismuth compounds, especially Colloidal Bismuth Subcitrate. It will be appreciated that two or more biologically active compounds can also be incorporated in a composition according to the invention.

Acid-resistant and release-limiting compounds to be used in the compositions according to the invention may be any one or a combination of the compounds known for this purpose in the art. Examples of known acid-resistant compounds are polymethacrylates, especially those known under the name of EUDRAGIT®-L and -S, hydroxypropyl methylcellulose phthalate, cellulose acetate phthalate, cellulose acetate trimellitate, polyvinyl acetate phthalate, and shellac. EUDRAGIT®-S has been found to be a preferable acid resistant compound.

Examples of known release-limiting compounds are the polymethacrylates known under the name EUDRAGIT®-RL,-RS and -NE, ethylcellulose and polyvinyl acetate, fatty acids such as stearic acid, fatty acid esters such as PRECIROL®, long chain aliphatic alcohols such as cetyl, stearyl, cetostearyl and myristyl alcohol, hydrogenated vegetable oils such as hydrogenated castor oil and hydrogenated cottonseed oil, waxes such as bees wax and distilled monoglycerides such as glyceryl monostearate. EUDRAGIT®-RS has been found to be a preferable release-limiting compound.

The percentage of the biologically active compound (w/w) in the solid dispersion can vary between 0.01 and 99%, depending on the the components used. When the biologically active compound is a corticosteroid such as beclomethasone 17,21-dipropionate its percentage (w/w) in the solid dispersion is preferably 0.1-40%, more preferably 5-15%. When the biologically active compound is a non-steroidal anti-inflammatory compound such as 5- or 4-amino-salicylic acid its percentage (w/w) in the solid dispersion is preferably 20-90%, more preferably 50-80%.

For the dispersion of the biologically active material in the acid-resistant or release-limiting substance, an organic or an aqueous solvent may be used.

As an organic solvent, methylene chloride has been found to be useful to disperse steroids, such as beclomethasone 17,21-dipropionate, in release-limiting substances. For the dispersion of said steroids in acid resistant sub-stances, and of non-steroidal anti-inflammatory compounds such as 5- or 4-amino-salicylic acid in both acid-resistant and release-limiting compounds, a mixture of about equal weights of ethanol and methylene chloride has been found to be very useful as the solvent.

When an aqueous solvent is employed to disperse a water-soluble biologically active substance, e.g. a therapeutic peptide, in the acid-resistant or release-limiting compound, aqueous polymeric dispersions have been found to be particularly useful as the aqueous solvent. Examples of these are aqueous dispersions of polymethacrylates such as EUDRAGIT®-L-30-D, EUDRAGIT®-RL-30-D, EUDRAGIT®-RS-30-D and EUDRAGIT®-NE-30,-40 or -50D, aqueous dispersions of submicron ethylcellulose spheres (AQUACOAT®) and aqueous dispersions of submicron cellulose acetate phthalate spheres (AQUATERIC®).

Examples of water-insoluble carriers to be used, singly or in combination, in the compositions according to the invention are powdered cellulose, microcrystalline cellulose, starches, colloidal silicon dioxide, bentonite, magnesium aluminum silicate and kaolin. Microcrystalline cellulose, such as is known under the proprietary name AVICEL®, has been found to be a preferred carrier.

The preferred mean size of the carrier particles is about 0.1 mm, and the preferred mean particle size of the granulate (carrier plus solid dispersion) is 0.1-2 mm, more preferably 0.5-1.2 mm.

The granulates according to the invention can be incorporated in any of the preparations for oral application known in the art, such as sachets, capsules and,- preferably, tablets, optionally also containing pharmaceutically acceptable excipients.

Tablets containing the granulates according to the invention have the practical advantages which are inherent to tablets in general, and additionally they have the advantage of being multi-particulate compositions, in that they disintegrate in the stomach, releasing the granules, which are small enough to leave the stomach rapidly and reliably. Alternatively, tablets containing the granulates according to the invention can be left to disintegrate in a small amount of water, rendering a homogeneous, drinkable dispersion.

It is known, e.g. from S.S. Davis et al., Gut (1986)27 886-892, that the gastric emptying of multiparticulate oral compositions resembles that of liquid dosage forms and differs from that of the bigger single units. While the intestinal transit time is approximately the same for the three dosage forms in both fasted and fed individuals, the gastric emptying of single units which do not disintegrate in the stomach tends to be slow and erratic, especially from a full stomach. Therefore there is an inherent advantage to multiparticulate dosage forms, above single-unit tablets.

The invention therefore also provides multiparticulate controlled-release oral compositions, in particular tablets, containing the granulates of the invention.

The following examples illustrate the invention.

Example 1

100 g of the acid-resistant EUDRAGIT®-S were dissolved in a mixture of 240.5 g methylene chloride and 240.5 g ethanol 96%. Then, 15 g beclomethasone 17,21-dipropionate were dissolved in the above EUDRAGIT®-S solution, and this solution was slowly (in 5 minutes) added to 385 g of the water-insoluble carrier AVICEL® PH 102 in a planet mixer, working at 60 revolutions per minute. The complete mixture was partially dried during one hour at 50°C, to a solvent content of 10-15%, and subsequently passed through an oscillating sieve of 1.0 mm apertures. The resulting granulate was further dried during 12 hours at 50-60°C, to constant weight. The batch of granules, now ready for further processing, had a total weight of about 500 grams. 50 grams of it had a free volume of 170 ml. Its residual content of ethanol and methylene chloride, determined by GLC, was 1.5% and 0.015%, respectively.

The granules had the following particle size distribution:

| Dimensions | | Percentage of total |
|---|---|---|
| > 1000 | μm | 0.9 |
| 1000-800 | " | 4.6 |
| 800-600 | " | 19.4 |
| 600-400 | " | 33.7 |
| 400-200 | " | 29.4 |
| < 200 | " | 12.0 |

Thermal analysis proved the material of the solid dispersion to be different from a simple mechanical mix of the same concentrations of beclomethasone 17,21-dipropionate crystals and EUDRAGIT®-S. While the mix demonstrated a sharp endotherm at 210°C which is characteristic of beclomethasone 17,21-dipropionate, the solid dispersion had no endotherm up to 230°C.

The content of beclomethasone 17,21-dipropionate in the granules was 3.0% w/w, as determined by HPLC.

Example 2

The disintegration adjuvants KOLLIDON®-CL and AVICEL® PH 102, both in the quantities of 28 g, were added to 500 g of the granules of Example 1 and mixed during 10 minutes. Subsequently 5.5 g of the lubricant magnesium stearate were added and mixed during a further 2 minutes. The mass was then fed to an excenter press tabletting machine, producing concave tablets having a diameter of 7 mm, a hardness of 9-11 kp and a disintegrating time (into the granules), in 0.1 N HCl at 37°C of 3-5 minutes. The tablets weighed 112.3 mg apiece, containing 3 mg beclomethasone 17,21-dipropionate and 20 mg EUDRAGIT®-S.

The dissolution rate of these tablets was tested according to USP-XXI at 37°C with the paddle stirred at 75 rpm. One tablet was put in a medium of 400 ml 0.1 N HCl + 2% CETOMACROGOL® 1000 (pH 1.3).

EP 0 375 063 B1

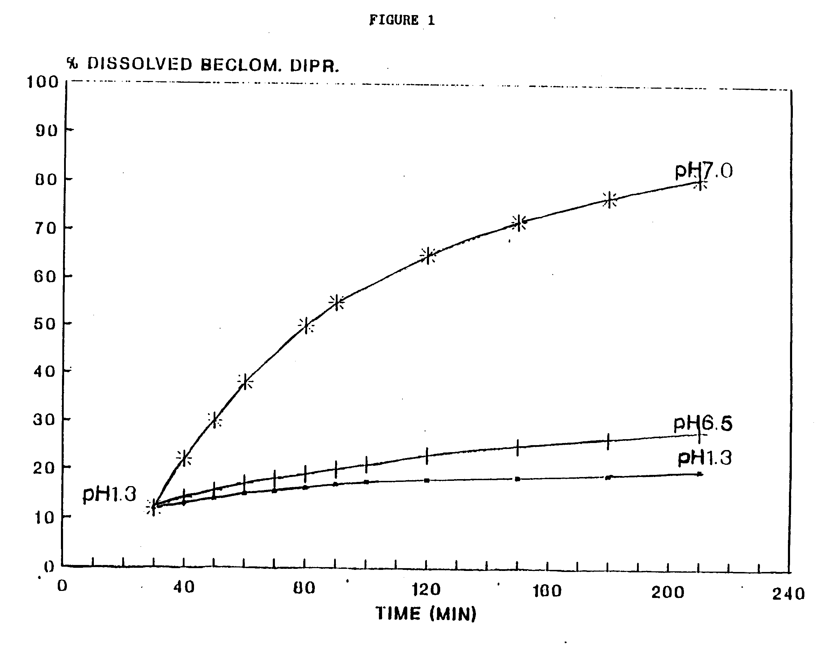

After 30 minutes the pH was raised to 6.5 or 7.0 by adding 45 and 50 ml respectively of 20% $Na_3PO_4.12H_2O$ and making up to 500 ml with water, after which the pH was checked and exactly adjusted using a few drops of 4N NaOH or 6N HCl. Beclomethasone 17,21-dipropionate was detected in samples of the medium taken at different time, whereby the HPLC method was used for quantification against a standard of 6 $\mu$g/ml beclomethasone 17, 21-dipropionate in phosphate buffer of pH 7.0 + 2% CETOMACROGOL® 1000. The results, as shown in figure 1, clearly demonstrate a pH-dependant release.

Example 3

62.5 g of the release-limiting compound EUDRAGIT®-RL were dissolved in 300 g methylene chloride. Then 7.5 g beclomethasone 17,21-dipropionate were dissolved in the above EUDRAGIT®-RL solution, and this solution was slowly (in 5 minutes) added to 180 g of AVICEL® PH 102 in a planet mixer, working at 60 revolutions per minute. The complete mixture was partially dried during one hour at 50°C, to a solvent content of 10-15%, and subsequently passed through an oscillating sieve of 1.0 mm apertures. The resulting granulate was further dried during 12 hours at 50-60°C, to constant weight of about 250 grams. 50 grams of it had a free volume of 198 ml. Its residual content of ethanol and methylene chloride, determined by GLC, was 0.012%.
The granules had the following particle size distribution:

| Dimensions | | Percentage of total |
|---|---|---|
| > 1000 | $\mu$m | 2.6 |
| 1000-800 | " | 21.3 |
| 800-600 | " | 30.2 |
| 600-400 | " | 26.0 |
| 400-200 | " | 14.9 |
| < 200 | " | 5.0 |

Thermal analysis proved the material of the solid dispersion to be different from a simple mechanical mix of the same concentrations of beclomethasone 17,21-dipropionate crystals and EUDRAGIT®-RS. While the mix demonstrated a sharp endotherm at 210°C which is characteristic of beclomethasone 17,21-dipropionate, the solid dispersion had no endotherm up to 230°C.
The content of beclomethasone 17,21-dipropionate in the granules was 2.75% w/w, as determined by HPLC.

Example 4

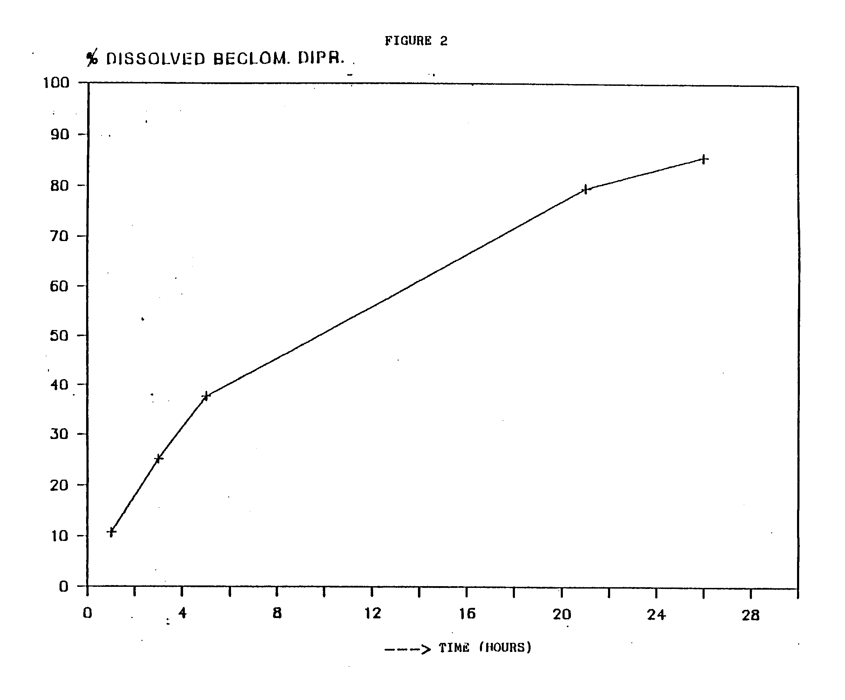

The disintegration adjuvants KOLLIDON®-CL and AVICEL® PH 102, both in the quantities of 12.5 g, were added to 222.5 g of the granules of Example 3 and mixed during 10 minutes. Subsequently 2.5 g of the lubricant magnesium stearate were added and mixed during a further 2 minutes. The mass was then fed to an excenter press tabletting machine, producing concave tablets having a diameter of 7 mm, a hardness of 6-7 kp and a disintegrating time (into the granules), in 0.1 N HCl at 37°C, of 3-5 minutes. The tablets weighed 117 mg apiece, containing 3 mg beclomethasone 17,21-dipropionate and 26 mg EUDRAGIT®-RL.
The dissolution rate of these tablets was tested in 300 ml pH 7 phosphate buffer + 1% CETOMACROGOL® 1000 in a beaker of 400 ml. The magnetic stirrer had a rate of 600 rpm. Beclomethasone 17,21-dipropionate was detected in samples of the medium taken at different time, whereby the HPLC method was used for quantification, against a standard of 6 $\mu$g/ml beclomethasone 17 21-dipropionate in phosphate buffer of pH 7.0 + 1% CETOMACROGOL® 1000. The results, as shown in figure 2, clearly demonstrate a sustained release.

Example 5

75 g of the release-limiting ethylcellulose and 75 g of hydrogenated castor oil were dissolved in 1175 g methylene chloride. Then 500 g 5-amino-salicylic acid were dispersed in the above solution, and this

EP 0 375 063 B1

dispersion was added in 2 minutes to 450 g of the water-insoluble carrier powdered cellulose in a planet mixer, working at 60 revolutions per minute. The complete mixture was subsequently passed through a sieve of 1.0 mm apertures. The resulting granulate was dried during 12 hours at 50-60 °C, to constant weight.

The dissolution rate of these granules was tested according to USP-XXI at 37 °C, with the paddle stirring at 100 rpm. 555 mg granules were put in 1000 ml of phosphate buffer medium of pH 7.5 to which was added 0.1% PLURONIC® F68. At time intervals up to 12 hours the absorbance at 326 nm was measured using a spectrophotometer equiped with a continuous-flow sampling system. The 5-amino-salicylic acid content was calculated using the absorbance value of a standard of 260 $\mu$g/ml of 5-amino-salicylic acid in phosphate buffer of pH 7.5, containing 0.1% PLURONIC® F68.

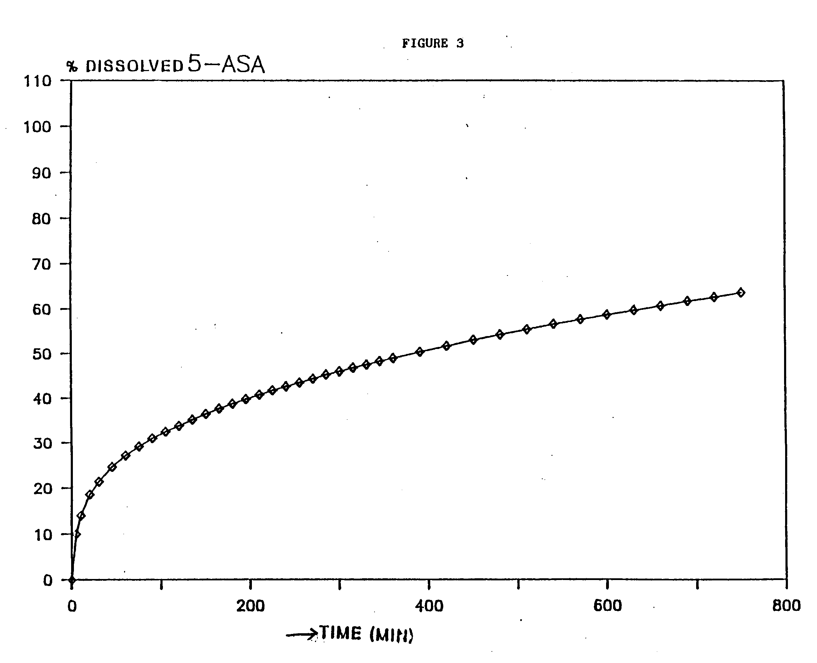

The results, as shown in figure 3, clearly demonstrate a sustained release from the granules.

**Claims**

1. Method for preparing a granulate for multi-particulate controlled-release oral compositions based on the concept of solid dispersion, whereby a biologically active substance is dispersed in an acid-resistant or release-limiting substance using the melting, the solvent or the melting-solvent method, characterized in that before the dispersion is solidified it is mixed with water-insoluble carrier particles whereafter the mixture is further processed according to granulating methods known in the art.

2. A granulate for multiparticulate controlled-release oral compositions comprising a biologically active substance in solid dispersion with an acid-resistant and/or release-limiting compound, characterized in that before the dispersion is solidified it is mixed with water-insoluble carrier particles.

3. A granulate according to claim 2, characterized in that the biologically active substance is a corticosteroid.

4. A granulate according to claim 3, characterized in that the corticosteroid is beclomethasone 17,21-dipropionate.

5. A granulate according to claim 2, characterized in that the biologically active substance is a non-steroidal anti-inflammatory drug.

6. A granulate according to claim 5, characterized in that the anti-inflammatory drug is 5-or 4-amino-salicylic acid, or a derivative thereof.

7. A granulate according to claim 2, characterized in that the biologically active substance is a bismuth compound.

8. A granulate according to claim 7, characterized in that the bismuth compound is Colloidal Bismuth Subcitrate.

9. A granulate according to any of claims 2-8, characterized in that the acid-resistant compound is EUDRAGIT®-S.

10. A granulate according to any of claims 2-8, characterized in that the release-limiting compound is EUDRAGIT®-PS.

11. A granulate according to any of claims 2-10, characterized in that the water-insoluble carrier is micro-crystalline cellulose.

12. A granulate according to any of claims 2-11, characterized in that the individual granules have a mean particle size of 0.1-2 mm.

13. A multiparticulate controlled-release oral composttion, containing a granulate according to any of claims 2-12.

6

**14.** A multiparticulate controlled-release oral composition according to claim 13, <u>characterized</u> in that it is a tablet.

**Patentansprüche**

**1.** Verfahren zur Herstellung eines Granulates für orale Multipartikelzubereitungen mit gesteuerter Freisetzung auf Basis des Konzepts der festen Dispersion, wobei eine biologisch aktive Substanz in einer säurebeständigen oder die Freisetzung begrenzenden Substanz unter Anwendung der Schmelz-, der Lösungsmittel- oder der Schmelz-Lösungsmittel-Methode dispergiert wird, dadurch gekennzeichnet, dass man die Dispersion, bevor man sie erstarren lässt, mit wasserunlöslichen Trägerpartikeln mischt, wonach das Gemisch gemäss Granulierungsmethoden, die dem Fachmann bekannt sind, weiterverarbeitet wird.

**2.** Granulat für orale Multipartikelzubereitungen mit gesteuerter Freisetzung, das eine biologisch aktive Substanz in fester Dispersion mit einer säurebeständigen und/oder die Freisetzung begrenzenden Verbindung aufweist, dadurch gekennzeichnet, dass man die Dispersion, bevor man sie erstarren lässt, mit wasserunlöslichen Trägerpartikeln mischt.

**3.** Granulat nach Anspruch 2, dadurch gekennzeichnet, dass die biologisch aktive Substanz ein Corticosteroid ist.

**4.** Granulat nach Anspruch 3, dadurch gekennzeichnet, dass das Corticosteroid Beclometason-17,21-dipropionat ist.

**5.** Granulat nach Anspruch 2, dadurch gekennzeichnet, dass die biologisch aktive Substanz ein nicht steroides entzündungshemmendes Arzneimittel ist.

**6.** Granulat nach Anspruch 5, dadurch gekennzeichnet, dass das entzündungshemmende Arzneimittel 5- oder 4-Amino-salicylsäure oder ein Derivat davon ist.

**7.** Granulat nach Anspruch 2, dadurch gekennzeichnet, dass die biologisch aktive Substanz eine Bismutverbindung ist.

**8.** Granulat nach Anspruch 7, dadurch gekennzeichnet, dass die Bismutverbindung Colloidal Bismuth Subcitrate ist.

**9.** Granulat nach einem der Ansprüche 2 bis 8, dadurch gekennzeichnet, dass die säurebeständige Verbindung EUDRAGIT®-S ist.

**10.** Granulat nach einem der Ansprüche 2 bis 8, dadurch gekennzeichnet, dass die die Freisetzung begrenzende Verbindung EUDRAGIT®-RS ist.

**11.** Granulat nach einem der Ansprüche 2 bis 10, dadurch gekennzeichnet, dass der wasserunlösliche Träger mnikrokristalline Cellulose ist.

**12.** Granulat nach einem der Ansprüche 2 bis 11, dadurch gekennzeichnet, dass die einzelnen Körner eine mittlere Partikelgrösse von 0,1 bis 2 mm haben.

**13.** Orale Multipartikelzubereitung mit gesteuerter Freisetzung, die ein Granulat nach einem der Ansprüche 2 bis 12 enthält.

**14.** Orale Multipartikelzubereitung mit gesteuerter Freisetzung nach Anspruch 13, dadurch gekennzeichnet, dass sie eine Tablette ist.

**Revendications**

**1.** Procédé de préparation d'un granulé pour des compositions orales multiparticulaires à libération contrôlée basées sur le principe de la dispersion solide, suivant lequel une substance biologiquement active est dispersée dans une substance résistante aux acides ou limitant la libération suivant le procédé par fusion, par solvant ou par fusion-solvant, caractérisé en ce qu'avant que la dispersion soit solidifiée, elle est mélangée avec des particules d'un excipient insoluble dans l'eau, après quoi le mélange est traité plus avant suivant des procédés de granulation connus dans la spécialité.

2. Granulé pour des compositions orales multiparticulaires à libération contrôlée comprenant une substance biologiquement active en dispersion solide avec un composé résistant aux acides et/ou limitant la libération, caractérisé en ce qu'avant que la dispersion soit solidifiée, elle est mélangée avec des particules d'un excipient insoluble dans l'eau.

3. Granulé suivant la revendication 2, caractérisé en ce que la substance biologiquement active est un corticostéroïde.

4. Granulé suivant la revendication 3, caractérisé en ce que le corticostéroïde est le 17,21-dipropionate de béclométhasone.

5. Granulé suivant la revendication 2, caractérisé en ce que la substance biologiquement active est un médicament anti-inflammatoire non-sté roïdien.

6. Granulé suivant la revendication 5, caractérisé en ce que le médicament anti-inflammatoire et l'acide 5- ou 4-aminosalicylique ou un dérivé de celui-ci.

7. Granulé suivant la revendication 2, caractérisé en ce que la substance biologiquement active est un dérivé du bismuth.

8. Granulé suivant la revendication 7, caractérisé en ce que le composé du bismuth est le sous-citrate de bismuth colloïdal.

9. Granulé suivant l'une quelconque des revendications 2 à 8, caractérisé en ce que le composé résistant aux acides est l'EUDRAGIT®-S.

10. Granulé suivant l'une quelconque des revendications 2 à 8, caractérisé en ce que le composé limitant la libération est l'EUDRAGIT®-RS.

11. Granulé suivant l'une quelconque des revendications 2 à 10, caractérisé en ce que l'excipient insoluble dans l'eau est la cellulose microcristalline.

12. Granulé suivant l'une quelconque des revendications 2 à 11, caractérisé en ce que les granules distincts ont une granulométrie moyenne de 0,1 à 2 mm.

13. Composition orale multiparticulaire à libération contrôlée, contenant un granulé suivant l'une quelconque des revendications 2 à 12.

14. Composition orale multiparticulaire à libération contrôlée suivant la revendication 13, caractérisée en ce qu'elle est un comprimé.

FIGURE 1

FIGURE 2

% DISSOLVED BECLOM. DIPR.

----> TIME (HOURS)

EP 0 375 063 B1

FIGURE 3

% DISSOLVED 5–ASA

EP 0 375 063 B1